# FASCICULE DE BREVET EUROPEEN

(11) **EP 2 379 477 B1**
(45) Date de publication et mention de la délivrance du brevet: **05.07.2017**
(21) Numéro de dépôt: 09807621.9
(22) Date de dépôt: 15.12.2009
(51) Int. Cl.: C07C 29/145, C07C 29/141, C07C 31/20

(54) **PROCEDE CATALYTIQUE DE FABRICATION DE COMPOSES DE TYPE DIOL, EN PARTICULIER MÉTHYL-2-PENTANE-2,4-DIOL**
KATALYTISCHES VERFAHREN ZUR HERSTELLUNG VON DIOLVERBINDUNGEN, INSBESONDERE 2-METHYL-2,4-PENTANDIOL
CATALYTIC METHOD FOR THE PRODUCTION OF DIOL COMPOUNDS, IN PARTICULAR 2-METHYL-2,4-PENTANEDIOL

(30) Priorité: 16.12.2008 FR 0858651
(43) Date de publication de la demande: 26.10.2011
(73) Titulaire: Rhodia Poliamida E Especialidades Ltda, Sao Paulo - SP (BR)
(72) Inventeur: ZIM, Danilo, 13090-600 CAMPINAS, SP (BR); MARION, Philippe, F-69390 Vernaison (FR)
(74) Mandataire: Ridray, Annabelle
(86) Numéro de dépôt international: PCT/IB2009/007760
(87) Numéro de publication internationale: WO 2011/077176

(56) Documents cités:
- DE-A1- 2 917 752
- JP-A- 9 020 703
- US-A- 5 663 452
- MOZINGO R.: "Catalyst, Raney Nickel, W-2" ORGANIC SYNTHESIS, vol. 21, 1941, page 15, XP002538184
- KANDEGEDARA A.ET AL.: "Noncomplexing tertiary amines as "Better" buffers covering the range of pH 3-11. Temperature dependance of their acid dissociation constants" ANALYTICAL CHEMISTRY, vol. 71, 1999, pages 3140-3144, XP002538185

## Description

La présente invention concerne un procédé catalytique pour la production à l'échelle industrielle d'un composé de type diol, tel que le 2-méthylpentane-2-4-diol, également appelé 2,4-hexylèneglycol, ou HGL, à partir d'un composé β hydroxy carbonylé, notamment le diacétone-alcool, ou DAA.

### ART ANTERIEUR

Le HGL est préparé industriellement par hydrogénation catalytique du DAA et le nickel de Raney est le catalyseur le plus utilisé, généralement dans des proportions importantes, notamment de 5-20% en poids par rapport au poids du DAA à hydrogéner.

Les conditions opératoires de la réaction d'hydrogénation du DAA en HGL en présence du catalyseur nickel de Raney induisent des problèmes techniques et économiques reconnus qui n'ont pas été surmontés à ce jour. En effet, dans les conditions de la réaction, le DAA peut se décomposer par rétro-aldolisation conduisant ainsi à la synthèse d'acétone puis d'isopropanol par hydrogénation. Le DAA peut également dans des conditions acides former de l'hexenol qui est ensuite hydrogéné en hexanol. Par ailleurs, ces conditions acides ont tendance à désactiver voire à solubiliser partiellement le catalyseur de la réaction.

Au surplus, il apparaît que le nickel de Raney doit être généralement lavé, notamment à l'eau distillée ou déminéralisée, pendant plusieurs jours, avant son utilisation dans la réaction d'hydrogénation de manière à diminuer sa basicité, afin d'accroître la sélectivité de la réaction. Cette étape est longue et contraignante et il serait fortement souhaitable de la supprimer, pour l'intérêt économique et industriel de cette réaction.
DE 2917752 décrit un procédé de préparation de diols 1,3 par hydrogénation catalysée par le Nickel de Raney, éventuellement prétraité par un composé tampon d'acide tartrique et dans lequel la réaction d'ydrogénation est maintenue à pH 4.

D'une manière plus générale, il existait un besoin de mettre au point un procédé industriel permettant d'hydrogéner des composés β hydroxy carbonylés possédant une fonction alcool sur le carbone en β de la fonction carbonyle, notamment une fonction aldéhyde, obtenus par des réactions d'aldolisation, tout en évitant une rétro-aldolisation de ces composés lors de la réaction catalytique d'hydrogénation.

### INVENTION

La demanderesse a trouvé un moyen tout a fait inattendu d'éviter les inconvénients mentionnés précédemment tout en obtenant une excellente sélectivité de la réaction d'hydrogénation sur les composé β hydroxy carbonylés, sans avoir par ailleurs besoin d'une longue étape de lavage du catalyseur au préalable.

La présente invention a ainsi pour premier objet un procédé pour la préparation d'un composé de formule (IV) par réaction d'hydrogénation catalytique d'un composé de formule (III), ledit composé de formule (III) étant obtenu par une réaction d'aldolisation baso catalysée, caractérisé en ce que ladite réaction d'hydrogénation est catalysée par un catalyseur de Raney qui a été prétraité par au moins un composé tampon permettant de maintenir ladite réaction d'hydrogénation à un pH compris entre 6 et 8, bornes incluses ;
les composés de formule (III) et (IV) sont les suivants : 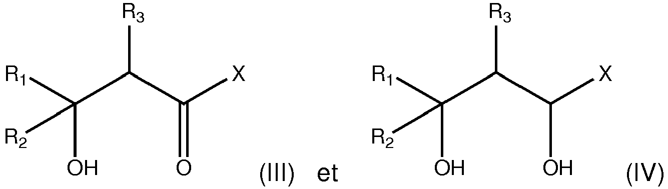 dans lesquelles :
- R¹ est un radical hydrocarboné comprenant 1 atome de carbone,
- R² est un radical hydrocarboné comprenant 1 atome de carbone,
- R³ est un atome d'hydrogène, et
- X est un groupe alkyle ;
le composé de formule (III) étant la diacétone-alcool (DAA) et le composé de formule (IV) étant le méthyl-2-pentane-2,4-diol (HGL).

La présente invention concerne ainsi un procédé pour la préparation du composé de formule (IV) par réaction d'hydrogénation catalytique du composé de formule (III) ci-dessus comprenant notamment au moins les étapes suivantes :
a) une étape de prétraitement du catalyseur de Raney par au moins un composé tampon permettant de maintenir un pH compris entre 6 et 8 lors de la réaction d'hydrogénation de l'étape b) ; et
b) une étape d'hydrogénation du composé de formule (III) pour former le composé de formule (IV) ; le pH du milieu réactionnel étant maintenant entre 6 et 8 lors de la réaction d'hydrogénation ; et
c) éventuellement une étape d'isolation du composé de formule (IV).

La présente invention concerne plus particulièrement un procédé pour la préparation de 2-méthylpentane-2-4-diol (HGL) par réaction d'hydrogénation catalytique de diacétone-alcool (DAA), caractérisé en ce que la réaction est catalysée par un catalyseur de Raney prétraité par un composé tampon permettant de maintenir ladite réaction à un pH compris entre 6 et 8, bornes incluses.

La présente invention a aussi pour objet le composé de formule (IV) susceptible d'être obtenu par le procédé mentionné précédemment.

Le radical hydrocarboné est le méthyle.

Le groupe alkyle, notamment pour la définition du radical X est un radical méthyle.

Comme explicité précédemment, le composé de formule (III) est un composé β hydroxy carbonylé possédant une fonction alcool sur le carbone en β de la fonction carbonyle, comme une fonction aldéhyde, cétone ou ester. Le composé β hydroxy carbonylé selon la présente invention est la diacétone-alcool (DAA).

Les composés de formule (III) est notamment obtenu par une réaction d'aldolisation baso catalysée entre un composé de formule (I) (R¹)(R²)C=O avec un composé de formule (II) R³-CH₂-COX. Le composé de formule (II) peut être une cétone ; c'est-à-dire un composé énolisable, possédant au moins un atome d'hydrogène sur le carbone en α du CO, dans la réaction d'aldolisation baso catalysée.

Le composé de formule (IV) est le méthyl-2 pentane diol-2,4 (HGL).

La présente invention consiste donc à effectuer un traitement du catalyseur par mise en présence de celui-ci avec un composé tampon, avant la conduite de la réaction d'hydrogénation. Au sens de l'invention, on appel tampon un composé capable de maintenir approximativement un pH entre 6 et 8 lors de la réaction d'hydrogénation.

La réaction d'hydrogénation des composés β hydroxy carbonylés est en elle-même bien connue de l'homme du métier.

La réaction de l'invention peut être conduite de manière continue ou discontinue. La réaction peut être conduite à une température comprise entre 70 et 150°C. La réaction peut être effectuée à une pression comprise entre 5 et 50 bar, notamment entre 10 et 25 bar. La durée de réaction, de manière à synthétiser une quantité optimale de composé de formule (IV) peut être comprise entre 5 minutes et 5 heures.

La réaction d'hydrogénation est notamment conduite en phase liquide. L'hydrogène peut notamment être dissout en totalité ou en partie dans le composé de formule (III).

La réaction peut notamment être mise en oeuvre dans un réacteur continu parfaitement agité pour la phase liquide. Le gaz peut être introduit soit par des systèmes de cannes plongeantes soit à l'aide de turbine auto-aspirante ou tout autre moyen par exemple avec une boucle de recirculation externe. On peut également utiliser un réacteur continu type piston tel qu'une colonne à bulles ou un réacteur à éjecteur venturi.

On utilise généralement de 0,1 à 20 % en poids de catalyseur par rapport au poids du composé de formule (III).

On utilise généralement de 0,01 à 2 % en poids de composé tampon par rapport au poids du composé de formule (III). On utilise généralement de 0,1 à 20 % en poids de composé tampon par rapport au poids du catalyseur.

Le catalyseur de Raney selon l'invention peut être un nickel de Raney, un cobalt de Raney ou un cuivre de Raney. Ces catalyseurs poreux sont bien connus et son généralement appelés catalyseur squelette ou catalyseur éponge. Le nickel de Raney est un catalyseur solide utilisé dans de nombreux procédés industriels. Il est notamment utilisé comme catalyseur hétérogène pour une grande variété de réactions de la chimie organique, le plus souvent pour des réactions d'hydrogénation. Le nickel de Raney est produit en traitant une poudre d'alliage nickel-aluminium par de la soude concentrée (Mozingo R, Catalyst, Raney Nickel, W-2, Organic Synthesis, vol. 21, 1941, page 15). Au cours de ce traitement appelé « activation », la majeure partie de l'aluminium de l'alliage est dissous avec en parallèle un fort dégagement d'hydrogène. La structure poreuse qui en résulte possède une surface spécifique très importante de l'ordre de 100 m²/g., ce qui contribue à son efficacité en catalyse.

Il existe de très nombreux tampons, utilisés seuls ou en combinaison, qui permettent de maintenir approximativement un pH entre 6 et 8 lors de la réaction d'hydrogénation du composé de formule (III) selon la présente invention. On entend par composé tampon selon l'invention, une solution contenant un acide et sa base conjuguée dont la propriété est de ne pas voir altérer de manière significative son pH en dépit de l'ajout d'une base ou d'un acide dans le milieu. La solution présente généralement un rapport de concentration acide/base comprises entre 0,01 et 100. On peut notamment mentionner les composés tampons organiques ou inorganiques cités dans les documents suivants : Good, N.E., et al. (1966) Hydrogen Ion Buffers for Biological Research. Biochemistry 5(2), 467-477; CRC Handbook of Chemistry and Physics, CRC Press Inc. David R. Lide, 1992-1993, 73eme édition, pages 8-37 - 8-42.

Le composé tampon selon l'invention est choisi dans le groupe comprenant :
- phosphate de potassium monobasique,
- phosphate de sodium monobasique,
- phosphate de lithium monobasique.

Il est à noter que l'on peut utiliser un ou plusieurs agents tampons lors de la réaction.

Le prétraitement peut être effectué en procédant à un mélange du catalyseur avec le composé tampon pendant une durée généralement comprise entre 1 heure et 24 heures. Ce mélange peut être effectué sans agitation particulière et à température ambiante. Le tampon peut se présenter sous forme liquide et le catalyseur peut se présenter sous forme de poudre.
Le catalyseur ainsi prétraité est alors mis en contact avec le milieu de la réaction de la présente invention.
En fin de réaction d'hydrogénation, on sort un mélange comprenant le composé de formule (III), le composé de formule (IV), le catalyseur et quelques sous produits. Le catalyseur peut être séparé des produits organiques à l'aide de tout moyen de séparation solide/liquide tel que par exemple un filtre ou un décanteur. Le liquide obtenu peut notamment être dirigé vers une colonne de distillation pour procéder à la séparation du composé de formule (III) et du composé de formule (IV).

Un langage spécifique est utilisé dans la description de manière à faciliter la compréhension du principe de l'invention. Il doit néanmoins être compris qu'aucune limitation de la portée de l'invention n'est envisagée par l'utilisation de ce langage spécifique. Des modifications, améliorations et perfectionnements peuvent notamment être envisagés par une personne au fait du domaine technique concerné sur la base de ses propres connaissances générales, l'invention étant définie seulement par les revendications jointes. Le terme et/ou inclut les significations et, ou, ainsi que toutes les autres combinaisons possibles des éléments connectés à ce terme.

D'autres détails ou avantages de l'invention apparaîtront plus clairement au vu des exemples donnés ci-dessous uniquement à titre indicatif.

### PARTIE EXPERIMENTALE

### Exemple 1

Dans un réacteur autoclave d'une capacité de 150 ml, on charge du DAA (80 g), du nickel de Raney (8 g) et éventuellement des quantités variables de tampon. Le catalyseur nickel de Raney peut être lavé à l'eau distillée pendant une semaine pour diminuer la basicité du catalyseur. C'est la procédure classiquement utilisée pour les catalyseurs nickel de Raney dans la réaction d'hydrogénation du DAA. Le catalyseur peut également être prétraité au préalable en le mélangeant avec le KH₂PO₄ pendant quelques heures.

L'autoclave est alors scellé, puis purgé avec de l'azote et de l'hydrogène. L'autoclave est alors mis sous pression avec de l'hydrogène à 20 bar et chauffé à une température de 100°C. L'agitation est effectuée par entrainement magnétique à 1500 tours par minute. Les données de la réaction telles que la température, la pression, la consommation d'hydrogène, la durée de la réaction et la conversion sont mesurées ou calculées pendant toute la réaction. A la fin de la réaction, le réacteur est refroidi et un échantillon est mesuré par une technique de chromatographie en phase gazeuse et caractérisé par un spectromètre de masse.

La sélectivité S d'une réaction chimique spécifie la quantité de produit désiré formé par rapport au nombre de moles consommées du réactif limitant. Elle indique si plusieurs réactions se produisent en parallèle, conduisant à des sous-produits non désirés, ou si la réaction recherchée est la seule à consommer du réactif.

Les résultats sont reportés sur le tableau 1 suivant :

**Tableau 1**

| **Essais** | **Catalyseur** | **Tampon** | **Conversion (%)** | **Sélectivité (%)** |
|---|---|---|---|---|
| C1 | Lavé | - | 98,29 | 96,48 |
| C2 | Non-lavé | Acide acétique (pH 7,0) | 99,96 | 40,89 |
| C3 | Non-lavé | KH₂PO₄ 5 % | 99,99 | 43,52 |
| 1 | Non-lavé | Pré-traitement KH₂PO₄ 5 % | 99,99 | 98,41 |
| 2 | Lavé | Pré-traitement KH₂PO₄ 5 % | 99,97 | 98,42 |

Le pourcentage de KH₂PO₄ est exprimé en poids par rapport au poids du catalyseur.

Dans l'essai C1, le catalyseur a été lavé pendant 7 jours à l'eau distillée. On observe une sélectivité insuffisante due à la formation d'IPA. Dans l'essai C2, le catalyseur n'a pas été lavé à l'eau au préalable mais a été prétraité avec une solution aqueuse contenant de l'acide acétique pour obtenir un pH de 7. On observe une sélectivité très médiocre due à une formation massive d'IPA. Dans l'essai C3, le catalyseur n'a pas été lavé ni prétraité. Du KH₂PO₄ a uniquement été ajouté dans le milieu de réaction. On observe encore une sélectivité très médiocre.

Dans l'essai 1, le catalyseur non lavé a été prétraité avec du KH₂PO₄. On observe une très bonne sélectivité. Dans l'essai 2, le catalyseur a été lavé et prétraité. On observe une sélectivité équivalente. Il apparaît ainsi que le prétraitement du catalyseur avec un tampon selon la présente invention permet non seulement d'obtenir une excellente sélectivité, mais également d'éliminer une étape de lavage du catalyseur au préalable.

### Exemple 2

La réaction est conduite de la même façon que dans l'exemple 1 mais en présence de 0,5 % en poids de nickel de Raney par rapport au poids de DAA.

Les résultats sont reportés sur le tableau 2 suivant :

**Tableau 2**

| **Essais** | **Catalyseur** | **Tampon** | **Conversion (%)** | **Sélectivité (%)** |
|---|---|---|---|---|
| C4 | Lavé | - | 95,56 | 93,58 |
| 3 | Lavé | Pré-traitement KH₂PO₄ 5 % | 95,17 | 96,34 |
| 4 | Lavé | Pré-traitement KH₂PO₄ 5 % | 84,70 | 96,54 |

Le pourcentage de KH₂PO₄ est exprimé en poids par rapport au poids du catalyseur.

On observe ainsi une très bonne sélectivité même en présence d'une faible quantité de catalyseur ; alors même qu'il est connu de l'art antérieur d'utiliser une quantité importante de catalyseur, de l'ordre de 5-20 % par rapport au DAA à hydrogéner.

## Revendications

1. Procédé pour la préparation d'un composé de formule (IV) par réaction d'hydrogénation catalytique d'un composé de formule (III), ledit composé de formule (III) étant obtenu par une réaction d'aldolisation baso catalysée, **caractérisé en ce que** ladite réaction d'hydrogénation est catalysée par un catalyseur de Raney qui a été prétraité par au moins un composé tampon permettant de maintenir ladite réaction d'hydrogénation à un pH compris entre 6 et 8, ledit composé tampon étant choisi parmi le phosphate de potassium monobasique, le phosphate de sodium monobasique et le phosphate de lithium monobasique ;
le composé de formule (III) étant la diacétone alcool (DAA) et le composé de formule (IV) étant le méthyl-2-pentane-2,4-diol (HGL).

2. Procédé pour la préparation du composé de formule (IV) par réaction d'hydrogénation catalytique du composé de formule (III), selon la revendication 1, comprenant notamment au moins les étapes suivantes :
a) une étape de prétraitement du catalyseur de Raney par au moins un composé tampon permettant de maintenir un pH compris entre 6 et 8 lors de la réaction d'hydrogénation de l'étape b) ; et
b) une étape d'hydrogénation du composé de formule (III) pour former le composé de formule (IV) ; le pH du milieu réactionnel étant maintenant entre 6 et 8 lors de la réaction d'hydrogénation ; et
c) éventuellement une étape d'isolation du composé de formule (IV).

3. Procédé selon l'une quelconque des revendications précédentes, **caractérisé en ce que** la réaction d'hydrogénation est conduite en phase liquide ; l'hydrogène étant dissout en totalité ou en partie dans le composé de formule (III).

4. Procédé selon l'une quelconque des revendications précédentes, **caractérisé en ce que** l'on utilise de 0,1 à 20 % en poids de catalyseur par rapport au poids du composé de formule (III).

5. Procédé selon l'une quelconque des revendications précédentes, **caractérisé en ce que** l'on utilise de 0,01 à 2 % en poids de composé tampon par rapport au poids du composé de formule (III).

6. Procédé selon l'une quelconque des revendications précédentes, **caractérisé en ce que** l'on utilise de 0,1 à 20 % en poids de composé tampon par rapport au poids du catalyseur.

7. Procédé selon l'une quelconque des revendications précédentes, **caractérisé en ce que** le catalyseur de Raney est un nickel de Raney, un cobalt de Raney ou un cuivre de Raney.

## Patentansprüche

1. Verfahren zur Herstellung einer Verbindung der Formel (IV) durch eine katalytische Hydrierungsreaktion einer Verbindung der Formel (III), wobei die Verbindung der Formel (III) durch eine basenkatalysierte Aldolisierungsreaktion erhalten wird, **dadurch gekennzeichnet, dass** die Hydrierungsreaktion von einem Raney-Katalysator katalysiert wird, der mit mindestens einer Pufferverbindung vorbehandelt wurde, wodurch die Hydrierungsreaktion bei einem pH-Wert zwischen 6 und 8 gehalten werden kann, wobei die Pufferverbindung aus monobasischem Kaliumphosphat, monobasischem Natriumphosphat und monobasischem Lithiumphosphat ausgewählt ist;
wobei die Verbindung der Formel (III) Diacetonalkohol (DAA) ist und die Verbindung der Formel (IV) Methyl-2-pentan-2,4-diol (HGL) ist.

2. Verfahren zur Herstellung der Verbindung der Formel (IV) durch eine katalytische Hydrierungsreaktion der Verbindung der Formel (III) nach Anspruch 1, das insbesondere mindestens die folgenden Schritte umfasst:
a) einen Schritt der Vorbehandlung des Raney-Katalysators mit mindestens einer Pufferverbindung, wodurch ein pH-Wert zwischen 6 und 8 während der Hydrierungsreaktion von Schritt b) aufrechterhalten werden kann; und
b) einen Schritt der Hydrierung der Verbindung der Formel (III) zur Herstellung der Verbindung der Formel (IV), wobei der pH-Wert des Reaktionsmediums während der Hydrierungsreaktion zwischen 6 und 8 gehalten wird; und
c) gegebenenfalls einen Schritt der Isolation der Verbindung der Formel (IV).

3. Verfahren nach einem der vorhergehenden Ansprüche, **dadurch gekennzeichnet, dass** die Hydrierungsreaktion in der Flüssigphase durchgeführt wird, wobei der Wasserstoff vollständig oder teilweise in der Verbindung der Formel (III) gelöst wird.

4. Verfahren nach einem der vorhergehenden Ansprüche, **dadurch gekennzeichnet, dass** man 0,1 bis 20 Gew.-% Katalysator, bezogen auf das Gewicht der Verbindung der Formel (III), verwendet.

5. Verfahren nach einem der vorhergehenden Ansprüche, **dadurch gekennzeichnet, dass** man 0,01 bis 2 Gew.-% der Pufferverbindung, bezogen auf das Gewicht der Verbindung der Formel (III), verwendet.

6. Verfahren nach einem der vorhergehenden Ansprüche, **dadurch gekennzeichnet, dass** man 0,1 bis 20 Gew.-% der Pufferverbindung, bezogen auf das Gewicht des Katalysators, verwendet.

7. Verfahren nach einem der vorhergehenden Ansprüche, **dadurch gekennzeichnet, dass** der Raney-Katalysator ein Raney-Nickel, ein Raney-Kobalt oder ein Raney-Kupfer ist.

## Claims

1. Method of preparing a compound of formula (IV) by a reaction of catalytic hydrogenation of a compound of formula (III), said compound of formula (III) being obtained by a reaction of base catalysed aldolization, **characterized in that** said hydrogenation reaction is catalysed by a Raney catalyst that was pretreated with at least one buffer compound enabling said hydrogenation reaction to be maintained at a pH between 6 and 8, said buffer compound being selected from monobasic potassium phosphate, monobasic sodium phosphate and monobasic lithium phosphate;
the compound of formula (III) being diacetone-alcohol (DAA) and the compound of formula (IV) being methyl-2 pentanediol-2,4 (HGL).

2. Method of preparing the compound of formula (IV) by a reaction of catalytic hydrogenation of the compound of formula (III), according to Claim 1, notably comprising at least the following steps:
a) a step of pretreatment of the Raney catalyst with at least one buffer compound for maintaining a pH between 6 and 8 during the hydrogenation reaction of step b); and
b) a step of hydrogenation of a compound of formula (III) to form a compound of formula (IV); the pH of the reaction mixture now being between 6 and 8 during the hydrogenation reaction; and
c) optionally a step of isolation of the compound of formula (IV).

3. Method according to either of the preceding claims, **characterized in that** the hydrogenation reaction is carried out in the liquid phase; the hydrogen being dissolved wholly or partly in the compound of formula (III).

4. Method according to any one of the preceding claims, **characterized in that** from 0.1 to 20 wt.% of catalyst is used relative to the weight of the compound of formula (III).

5. Method according to any one of the preceding claims, **characterized in that** from 0.01 to 2 wt.% of buffer compound is used relative to the weight of the compound of formula (III).

6. Method according to any one of the preceding claims, **characterized in that** from 0.1 to 20 wt.% of buffer compound is used relative to the weight of the catalyst.

7. Method according to any one of the preceding claims, **characterized in that** the Raney catalyst is a Raney nickel, a Raney cobalt or a Raney copper.
